# EUROPEAN PATENT SPECIFICATION

(11) **EP 3 554 308 B1**
(45) Date of publication and mention of the grant of the patent: **04.05.2022**
(21) Application number: 17811322.1
(22) Date of filing: 12.12.2017
(51) Int. Cl.: A45D 2/00

(54) **COSMETIC PRODUCT REFILL IN THE FORM OF AN AQUEOUS COMPOSITION FOR A HAIR TREATMENT DEVICE**
NACHFÜLLUNG EINES KOSMETISCHEN PRODUKTS IN FORM EINER WÄSSRIGEN ZUSAMMENSETZUNG FÜR EINE HAARBEHANDLUNGSVORRICHTUNG
COSMETIC PRODUCT REFILL IN THE FORM OF AN AQUEOUS COMPOSITION FOR A HAIR TREATMENT DEVICE

(30) Priority: 15.12.2016 FR 1662518
(43) Date of publication of application: 23.10.2019
(73) Proprietor: L'OREAL, 75008 Paris (FR); SEB S.A., 69130 Ecully (FR)
(72) Inventor: WOODLAND, Frédéric, 93400 Saint-Ouen (FR); NUZZO, Stefania, 93400 Saint-Ouen (FR); LAPIZE, Sandy, 93400 Saint-Ouen (FR); CHAMPEAUX, Mélissa, 93500 Saint-Ouen (FR); VIC, Gabin, 93400 Saint-Ouen (FR)
(74) Representative: Nony
(86) International application number: PCT/EP2017/082360
(87) International publication number: WO 2018/108872

(56) References cited:
- EP-A1- 3 039 988
- FR-A1- 3 022 781

## Description

The present invention relates to cosmetic product refills for hair treatment devices, and more particularly, but not exclusively, those for devices for shaping the hair, notably intended for straightening, curling or crimping the hair.

The invention also relates to devices provided with such a refill and to methods for treating the hair.

### Background

Usually, hair straighteners consist of two arms that are connected together with the aid of a hinge which makes it possible to open and close said arms, and of at least one heating element disposed on the arms. During operations of styling a lock of hair, said lock is introduced between the two arms in the open position and then the two arms are closed manually over the lock of hair. The latter is then subjected to the heat output by the heating element, until the two arms are opened and the lock of hair is removed.

The application WO 2009/078046 describes a hairstyling appliance comprising two arms that are connected together so as to allow the appliance to be opened and closed, at least one heating member and at least one seat for accommodating a hair treatment device, the latter allowing a haircare product to be dispensed during operation. The hair treatment device comprises a support material impregnated with a haircare product and suitable for a single use.

EP3039 988 discloses a refill for a hair treatment device.

WO 2009/015027 and US 2009/0025247 disclose a hair straightening device that makes it possible to apply a haircare product by contact with the hair. The haircare product to be applied is contained in a removable refill for the application thereof. Said removable refill comprises a reservoir containing the haircare product in a gelled form, and orifices for dispensing and applying the product, said orifices being made directly through a wall of the reservoir. The refill is introduced into a housing disposed on one of the two arms of the hair straightener, by sliding.

The application WO 2013/045331 likewise discloses an applicator for applying a care substance to the hair, comprising a cartridge of care substance held in a housing of the applicator. The cartridge comprises a porous support saturated with the care substance.

The application WO 2013/090896 relates to a hair treatment appliance, in particular a hairstyling appliance, comprising a treatment agent holder, notably a saturated porous material, to be applied to the hair or the skin. The holder is fixed to an accessory, which is itself mounted in a removable manner on a hair treatment appliance. The holder is T-shaped, allowing it to be mounted in a complementary groove in the accessory.

There is a need to further improve devices for applying a haircare product so as to ensure adequate application of cosmetic product, notably when the latter is in the form of an aqueous composition.

### Summary of the invention

A subject of the invention is thus a cosmetic product refill according to claim 1.

Such a pairing of density of the applicator member/viscosity of the aqueous composition makes it possible to have a quantity of cosmetic product on the hair which is both sufficient and sufficiently consistent during uses.

Preferably, the cosmetic product refill according to the invention has one or more of the following features, on their own or in combination:
- the applicator member is porous;
- the applicator member has a density of between 0.11 and 0.15 g/cm³ and the aqueous composition has a viscosity greater than 20 centipoise and/or less than 25 centipoise, at 22°C;
- the applicator member is a felt, the fibers of the applicator member preferably being oriented parallel or perpendicularly to the surface intended to be in contact with the hair to be treated, and notably the fibers are oriented in a longitudinal direction of the applicator member, parallel to the surface intended to be in contact with the hair to be treated;
- the applicator member, notably the felt, if appropriate, is made of polyethylene, of polypropylene, or of a mixture of polyethylene and polypropylene, the mixture preferably comprising 50% polyethylene and 50% polypropylene;
- the applicator member is soaked with aqueous composition with an initial degree of filling less than or equal to 90%, preferably less than or equal to 80%, more preferably less than or equal to 70%;
- the applicator member is soaked with aqueous composition with an initial degree of filling greater than or equal to 40%, preferably greater than or equal to 50%, more preferably greater than or equal to 60%;
- the applicator member is secured to a reservoir of aqueous composition to be applied to the hair to be treated, the reservoir preferably being made of the same material as the applicator member, more preferably with a lower density;
- the applicator member and the reservoir, if appropriate, are saturated with a volume of aqueous composition greater than 6 ml, preferably greater than 10 ml, and/or less than 14 ml, preferably less than 11 ml;
- the applicator member has an elongate shape, with a rectangular or cruciform cross section, with a flat, pointed or rounded end that is intended to be in contact with the hair to be treated, and/or with one or two bevels;
- the refill comprises a body delimiting a cavity that is formed at least partially by two opposite walls and a bottom and opens toward the outside by way of an opening, said applicator member being partially received in said cavity and extending partially out of the cavity, through the opening;
- the aqueous composition comprises one or more non-silicone surfactants and/or one or more silicone compounds, the non-silicone surfactant(s) being chosen from anionic, cationic, nonionic or amphoteric surfactants, and preferably from cationic surfactants;
- the non-silicone surfactant(s) and/or the silicone compound(s) are present in the aqueous composition in a content of between 0.01 and 10%, better still between 0.05 and 5% by weight, relative to the total weight of the aqueous composition;
- the aqueous composition comprises one or more fatty substances and/or one or more non-silicone polymers, the fatty substance(s) preferably being chosen from fatty alcohols, oils, and plant waxes, and the non-silicone polymer(s) preferably being chosen from cationic non-silicone polymers;
- the aqueous composition is in the form of an emulsion;
- the aqueous composition appearing to the eye in a single visible phase.

According to another aspect, the invention relates to a cosmetic product refill article, comprising a closed package and at least one cosmetic product refill as described above, in the package.

The invention also relates to a hair treatment device, comprising:
- at least one holder, preferably an arm comprising a housing,
- a cosmetic product refill as described above in all its combinations, which is disposed in a removable manner in the housing.

Preferably, the device comprises at least one heating element.

Preferably, the refill is disposed in a removable manner in a housing of the arm.

The hair treatment device being a hair straightener, notably a straightening iron.

Preferably, the hair treatment device comprises two arms that are able to move relative to one another between a moved-together configuration for treating the hair and a spaced-apart configuration for inserting hair to be treated between said arms.

According to one variant, at least one of the arms, preferably each arm, comprises a heating element for the hair to be treated.

According to yet another aspect, the invention relates to a ready-to-assemble hair treatment assembly, comprising:
- a hair treatment device, comprising at least one arm provided with a heating element for the hair, the hair treatment device comprising, on one of these arms, a receiving housing suitable for receiving a cosmetic product refill as described above, and
- a cosmetic product refill article as described above, in all the combinations thereof.

According to another aspect, the invention also relates to a method for treating the hair, comprising the step of applying a cosmetic product as described above to the hair with the aid of the device according to the invention.

Preferably, the treatment is carried out by moving the device along the lock of hair, the arms of said device being held in the moved-together configuration during said movement.

The invention may be understood better from reading the following detailed description of nonlimiting illustrative embodiments thereof and from studying the appended drawing, in which:
- Figure 1 shows a perspective view of an example of a hair treatment device,
- Figure 2 is a cross-sectional view on II-II in figure 1,
- Figure 3 illustrates, in cross section, examples of applicator members that can be employed in the hair treatment device in figure 1,
- Figure 4 illustrates tests of the quantity of cosmetic product delivered by different types of applicator member; and
- Figure 5 schematically illustrates a ready-to-assemble hair treatment assembly.

In the rest of the description, identical elements or elements having identical functions bear the same reference signs. In order to make the present description concise, they are not described for each of the figures, only the differences between the different examples being described.

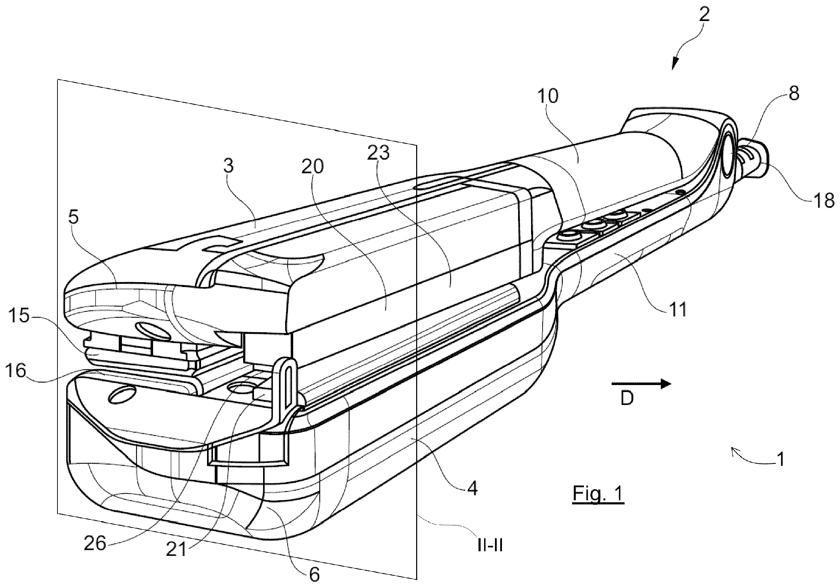

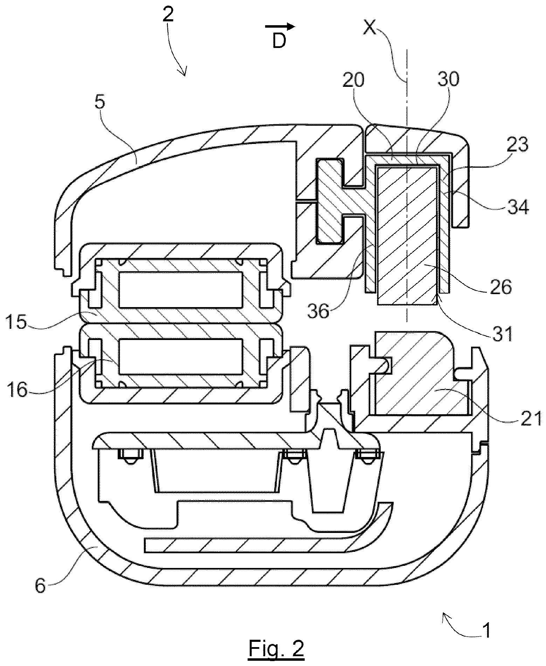

Figures 1 and 2 show the handpiece 2 of an example of a hair treatment device 1.

This handpiece 2 has two jaws 3 and 4 that are able to move with respect to one another between a spaced-apart configuration (not shown) for the introduction of a lock of hair between said jaws, and a moved-together configuration for treating the lock of hair.

The jaws 3 and 4 are carried by an upper arm 5 and a lower arm 6, respectively, which, in the example in question, are connected together at one end by an articulation 8, the handpiece 2 thus forming tongs.

The upper arm 5 and lower arm 6 each preferably have a total length of between 22 cm and 37 cm, preferably 31 cm, and define, between the articulation 8 and the jaws 3 and 4, respective half-handles 10 and 11 on which the user can press in order to move the jaws 3 and 4 together.

An elastic return member (not visible) is preferably provided to return the jaws 3 and 4 to a spaced-apart configuration, this elastic return member being for example a spring disposed around a pin of the articulation 8.

The invention is not limited to a particular manner of connecting the upper arm 5 and lower arm 6 together and the jaws 3 and 4 may be rendered able to move in some other way without departing from the scope of the present invention. However, the presence of an articulation is largely preferred for the ergonomics it provides.

The jaws 3 and 4 define between them a region for treating the hair, said region being intended to receive a lock of hair to be treated, the handpiece 2 being moved along said lock during the treatment, for example in the direction from the root to the end of the hair.

In the example in question, the handpiece 2 is configured to apply a cosmetic product, to treat the hair by way of steam and then to carry out a heat treatment of the hair by contact with two hot surfaces of heating elements 15 and 16 that are carried by the upper arm 5 and the lower arm 6, respectively.

The direction D of movement of the handpiece 2 over the hair, illustrated in figure 1, is preferably substantially perpendicular to the upper arm 5 and lower arm 6.

The handpiece 2 is connected by a line, in the example in question, to a base station (not shown) that is fixed during the treatment and is connected to the mains.

This base station provides electric power to the handpiece 2 and also its supply of water for generating steam, and may also carry out additional functions of processing electrical signals received from the handpiece 2. The line 18 which connects the handpiece 2 to the base station may thus comprise various electrical conductors and a water supply pipe.

A user interface (not shown in the figures) may be present on the handpiece 2 so as to give the user the option for example of starting up certain components thereof.

The cosmetic product is applied by a refill 20 carried by one of the two arms, 5 or 6, in this case the upper arm 5, which comes into contact with a pressing element 21. The latter may be removable.

The refill 20 comprises a body 23 and an applicator member 26 disposed so as to come into contact with the hair extending through the treatment region.

The refill 20 may be fastened to the arm 5 or 6 by any means, notably, as illustrated in figure 2, by sliding a rib of the body 23 into a groove with a complementary shape.

As illustrated in figure 2, the body 23 has a cavity 30 that opens toward the outside by way of an opening 31 into which the applicator member 26 is inserted, along an insertion axis X, while the refill is being manufactured. The body may comprise coupling reliefs that extend into the cavity 30.

The body 23 substantially has an elongate shape along a longitudinal axis. In cross section, the body 23 is U-shaped, as illustrated in figure 2.

As illustrated in figure 2, the applicator member 26 may be in the form of a rectangular parallelepiped. The applicator member 26 thus has a substantially flat face that is intended to be in contact with the hair to be treated and is parallel to the opposite surface of the applicator member 26. The dimensions of the applicator member 26 are thus, for example, 8 mm x 20 mm x 90 mm, the face intended to be in contact with the hair having dimensions of 8 mm x 90 mm.

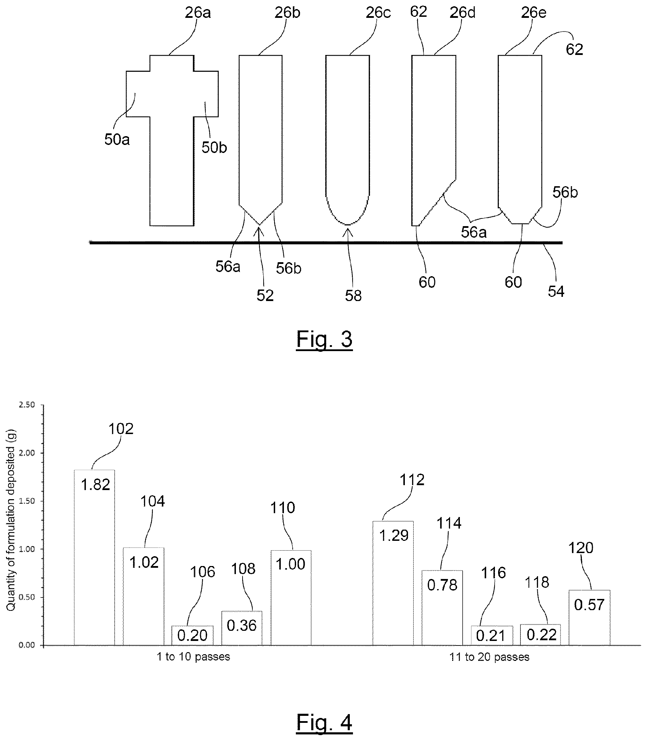

However, figure 3 illustrates possible variants of the applicator member 26. The applicator member 26a thus has a cruciform cross section, this applicator member 26a having, compared with the applicator member 26 in figure 2, two parallelepipedal longitudinal ribs 50a, 50b.

The applicator member 20b for its part has, compared with the applicator member 26 in figure 2, a point 52 intended to be in contact with the hair 54 to be treated. The point 52 is formed by two bevels 56a, 56b with opposite orientations.

The applicator member 26c has, in cross section, an ogival shape, the rounded end 58 of the ogive being intended to be oriented toward the hair 54.

The applicator member 26d has, compared with the applicator member 26b, a single bevel 56a. The applicator member 26d thus has a surface 60, intended to be in contact with the hair 54, that is parallel to the opposite surface 62 and has limited dimensions.

Finally, the applicator member 26e has two opposite bevels 56a, 56b that are connected by a central portion 60, intended to be in contact with the hair 54, which extends substantially parallel to the opposite surface 62 of the applicator member 26e.

Other applicator member shapes may also be envisioned, notably by combining the variants in figure 3.

The applicator member may be made for example of polyethylene, of polypropylene or of a mixture of polyethylene and polypropylene. The mixture may notably comprise 50% polyethylene and 50% polypropylene.

The applicator member may be made of felt. A felt is, preferably, a nonwoven material obtained by agglutinating natural or synthetic fibers.

The fibers of the felt may be oriented parallel to the surface of the lock of hair 54 to be treated, the fibers preferably being oriented substantially in the direction D of passage over the hair. In one variant, the fibers of the felt of the applicator member are oriented perpendicularly to the surface of the lock of hair 54 to be treated, i.e. perpendicularly to the direction D and to the longitudinal direction of the applicator member 26.

The applicator member may comprise at least two parts having different fiber densities and/or fiber orientations. Notably, a part with a lower fiber density may act as a reservoir, while the other part, with a higher fiber density, allows more homogeneous application of cosmetic product to the lock of hair to be treated. In this way, the variation in quantity of product delivered onto one and the same lock is reduced, depending on the position on the lock.

The applicator member is for example initially saturated with a volume of cosmetic product greater than 6 ml, preferably greater than 10 ml and/or less than 14 ml, preferably less than 11 ml. The applicator member has for example a density greater than 0.11 g/cm³ and/or less than 0.25 g/cm³.

The cosmetic product is in this case an aqueous composition, notably in the form of an emulsion.

An aqueous composition is understood to be a composition comprising at least 5% by weight water.

Preferably, the composition comprises at least 20% by weight water, more preferably at least 50% by weight water, even more preferably at least 90% by weight water, relative to the total weight of the composition.

The emulsion is preferably direct.

The solids content of the emulsion is preferably less than 10% by weight.

The aqueous composition according to the invention preferably comprises one or more non-silicone surfactants and/or one or more silicone compounds.

The surfactants that are usable in the composition may be anionic, cationic, nonionic or amphoteric, and preferably cationic.

Non-silicone surfactants are understood to be surfactants that do not contain atoms of silicon in their structure.

In one particular embodiment of the invention, the composition comprises one or more cationic surfactants chosen from quaternary ammonium salts.

When they are present, the non-silicone surfactants are preferably present in the aqueous composition in a content ranging from 0.01 to 10%, better still from 0.05 to 5% by weight, relative to the total weight of the aqueous composition.

Silicone is understood to mean compounds comprising one or more atoms of silicon in their structure.

The cosmetic composition according to the invention may comprise one or more silicones, which may be solid or liquid, and volatile or non-volatile.

The silicones that may be used may be soluble or insoluble in the composition according to the invention; they may be in the form of oil, wax, resin or gum; silicone oils and gums are preferred.

When they are present, the silicones are preferably present in the aqueous composition in a content ranging from 0.01 to 10%, better still from 0.05 to 5% by weight, relative to the total weight of the aqueous composition.

The aqueous composition according to the invention may also comprise one or more ingredients chosen from non-silicone fatty substances, non-silicone polymers, notably cationic, nonionic, anionic or amphoteric non-silicone polymers, UV-screening agents, colorants, pigments, preservatives and fragrances.

Preferably, the aqueous composition according to the invention comprises one or more non-silicone fatty substances and/or one or more non-silicone polymers. More preferably, the aqueous composition according to the invention comprises one or more non-silicone fatty substances chosen from fatty alcohols, natural or synthetic oils or plant waxes and/or one or more cationic non-silicone polymers.

If they are present, the non-silicone fatty substances and/or the non-silicone polymers are preferably present in a content ranging from 0.05 to 10%, better still from 0.1 to 5% by weight, relative to the total weight of the aqueous composition.

The aqueous composition has a viscosity greater than or equal to 4 centipoise, preferably greater than or equal to 5 centipoise.

The aqueous composition has a viscosity of between 4 and 250 centipoise, preferably between 4 and 100 centipoise The viscosity of the aqueous composition is measured at a pressure of 1.01325 10⁵ Pa, at a temperature of 22°C and at a shear rate of 1 s⁻¹. This viscosity may be measured using a cone/plate viscometer, notably a Haake R600 rheometer or the like.

After numerous tests, the inventors have found, surprisingly, that the combination of a density of the applicator member as described above and a viscosity of the aqueous composition as described above makes it possible to deliver a satisfactory quantity of aqueous composition onto locks of hair to be treated, at least during the first passes of the applicator member over the locks of hair to be treated, in order to obtain a satisfactory cosmetic effect of the aqueous composition on the hair. This is true notably for the first 70 passes, preferably for the first 75 passes, of the applicator member over locks of hair to be treated, these numbers corresponding to the treatment of an entire head. The passes of the applicator member are understood here as being passes at a substantially constant speed of 15 s over locks with a length of 27 cm and a width of 10.5 cm, with a force of 3 N being applied to the applicator member in the direction of the lock of hair to be treated.

The initial quantity of cosmetic product present in the applicator member preferably corresponds to an initial degree of filling of the applicator member less than or equal to 90%, preferably less than or equal to 85%, and better still less than 80%, and greater than or equal to 40%, preferably greater than or equal to 50%, and better still greater than or equal to 60%. Specifically, the inventors have found, surprisingly, that, when the applicator member is not completely saturated with cosmetic product but rather has a degree of filling as indicated above, the quantity of cosmetic product delivered on each pass over the hair to be treated exhibits less significant variations. On account of the use of refills according to the invention, it is possible to obtain a more regular, notably more homogeneous cosmetic effect on all of a user's hair. Of course, the consistency of the quantity of cosmetic product implies that the conditions of application of the cosmetic product, notably the speed of passage and the force of application, are kept substantially constant over the entire period of use of the hair treatment device.

The "initial quantity" is understood here to be the quantity of cosmetic product present in the applicator member before the first pass over the hair to be treated.

The "degree of filling of the applicator member" is understood here to be the ratio between the mass of cosmetic product effectively present in the applicator member and the maximum mass of cosmetic product with which the applicator member can be saturated. This degree of filling is measured at atmospheric pressure of 1.01325 10⁵ Pa and a temperature of 22°C.

By way of example, figure 4 illustrates the results of tests of the application of particular aqueous compositions to strips of paper with the aid of different applicator members.

### Examples:

The following compositions A, B and C were prepared from the ingredients indicated in the tables below. The concentrations are expressed as weight percentages of active material in the composition.

| Ingredients | A |
|---|---|
| Stearyl alcohol | 1.5 |
| PEG-40 Hydrogenated castor oil | 0.8 |
| Dimethicone | 2.5 |
| Isopropyl alcohol | 0.18 |
| Amodimethicone | 0.575 |
| Behentrimonium chloride | 0.79 |
| Laureth-4 | 0.145 |
| Laureth-23 | 0.105 |
| Fragrance, preservative | qs |
| Water | qs 100 |

The viscosity of this aqueous composition A is 22 centipoise at 22°C.

| Ingredients | B |
|---|---|
| Trideceth-5 | 0.81 |
| Trideceth-10 | 0.2025 |
| Polyquaternium-37 | 0.75 |
| Glycerol | 0.4725 |
| PPG-1 Trideceth-6 | 0.105 |
| PEG-40 Hydrogenated castor oil | 0.8 |
| Amodimethicone | 2.025 |
| Propylene glycol dicaprylate/dicaprate | 0.525 |
| Fragrance, preservative | qs |
| Water | qs 100 |

The viscosity of this aqueous composition B is 280 centipoise at 22°C.

| Ingredients | c |
|---|---|
| PEG/PPG/Polybutylene glycol-8/5/3 glycerol | 0.2 |
| Polyquaternium-37 | 0.5 |
| Polyquaternium-11 | 0.2 |
| Glycerol | 3 |
| PPG-1 Trideceth-6 | 0.07 |
| PEG-40 Hydrogenated castor oil | 1.5 |
| Amodimethicone | 0.69 |
| Propylene glycol dicaprylate/dicaprate | 0.35 |
| Fragrance, preservative | qs |
| Water | qs 100 |

The viscosity of this aqueous composition C is 87 centipoise at 22°C.

The test to which the different samples of applicator members saturated with cosmetic product are subjected consists in:
- carrying out ten passes of 15 seconds each of the applicator member over strips of paper of 75 g/m² IQ Appeal^{®} from Mondigroup, each strip measuring 27 cm in length and 10.5 cm in width, a force of 3 N being applied to the applicator member, in the direction of the strip of paper, so as to deliver a first quantity of aqueous composition onto the ten strips. This first quantity may be determined for example by weighing the applicator member before and after the ten passes, the total quantity of aqueous composition deposited on the ten strips then corresponding to the difference between the masses measured;
- then, carrying out ten more passes of 15 seconds of the applicator member over new strips of paper of 75 g/m² IQ Appeal^{®} from Mondigroup, each strip again measuring 27 cm in length and 10.5 cm in width, a force of 3 N being applied to the applicator member, in the direction of the strip of paper, so as to deliver a second quantity of aqueous composition.

It should be noted here that it is important to saturate the strips along their entire length. In other words, the applicator member is moved over the strip at a speed of around 1.8 cm/s.

In all cases, the applicator members tested have a parallelepipedal shape with fibers oriented substantially in a longitudinal direction of the applicator member, parallel to the surface to which the aqueous composition is applied. They are constituted of a mixture of 50% polyethylene and 50% polypropylene.
1^{st} sample tested:
   The first sample tested comprises an applicator member with a density of 0.1 g/cm³ saturated to 80% with Aqueous composition A described above.
2^{nd} sample tested:
   The second sample differs from the first only by way of the density of the applicator member. It is equal to 0.13 g/cm³.
3^{rd} sample tested:
   The third sample differs from the first and second only by way of the density of the applicator member, which is equal to 0.35 g/cm³.
4^{th} sample tested:
   The fourth sample comprises an applicator member with a density of 0.13 g/cm³ saturated to 80% with Aqueous composition B described above.
5^{th} sample tested:
   This final sample comprises an applicator member with a density of 0.10 g/cm³ saturated to 80% with Aqueous composition C described above.

Bars 102, 104, 106, 108 and 110 in figure 4 indicate the first quantity of aqueous composition deposited with the first, second, third, fourth and fifth samples, respectively, and correspond to the following values: 1.82 g, 1.02 g, 0.20 g, 0.36 g and 1.00 g.

Bars 112, 114, 116, 118 and 120 in figure 4 indicate the second quantity of aqueous composition deposited with the first, second, third, fourth and fifth samples, respectively, and correspond to the following values: 1.29 g, 0.78 g, 0.21 g, 0.22 g and 0.57 g.

It has been found that the best cosmetic results on hair, notably a better cosmetic feel and better homogeneity of deposition, were obtained when the two following criteria are met:
- a deposition of aqueous composition per pass over strips of paper in the range from 0.5 g to 1.2 g;
- the absolute value of the difference between the first quantity and the second quantity of aqueous composition is less than 0.5 g.

It was found that the first sample delivers too large a quantity of cosmetic product, while the third and fourth samples, for their part, deliver too small a quantity of cosmetic product. Therefore, these samples are unsuitable for the desired application.

By contrast, the quantities of cosmetic product delivered by the second and fifth samples are in the desired range. The difference between the first and second quantities of cosmetic product for these two samples is 0.24 g and 0.43 g, respectively. These two samples therefore meet the two sought-after criteria and can thus be employed in the scope of the desired application.

The invention is not limited to the illustrative embodiments that have just been described, the features of which may be combined with one another within variants that are not illustrated.

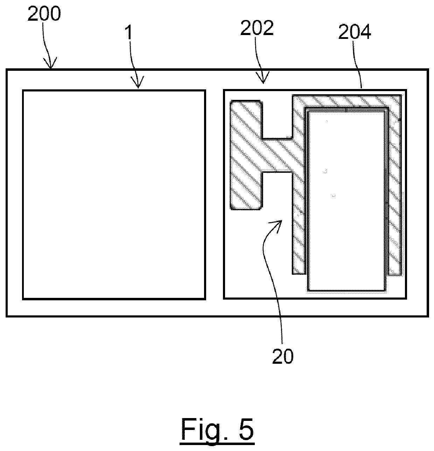

Thus, the invention relates, for example, to a ready-to-assemble assembly 200 as illustrated schematically in figure 5. This ready-to-assemble assembly 200 comprises a hair treatment device 1 and a cosmetic product refill article 202. This article comprises in this case a cosmetic product refill 20 in a package 204 which is closed so as to avoid, notably, any evaporation of the cosmetic product from the applicator member. In a variant, the package 204 may comprise a plurality of cosmetic product refills 20. In this case, it is preferred for the package to be able to be reclosed so that the other cosmetic product refills do not dry out when a refill is withdrawn from the package. In a variant, in this case, each individual refill is preserved in an individual package, inside a package common to the multiple refills.

Moreover, other aqueous compositions can be employed, as long as their viscosity is between 5 and 250 centipoise, and preferably 4 to 100 centipoise, at 22°C and 1.01325 10⁵ Pa at a shear rate of 1 s⁻¹.

## Claims

1. A cosmetic product refill (20) for a hair treatment device (1), comprising an applicator member (26), preferably a porous applicator member (26), saturated with an aqueous composition, wherein the applicator member has a density of between 0.11 and 0.25 g/cm³ and **characterized in that** the aqueous composition has a viscosity of between 4 and 250 centipoise: preferably between 4 and 100 centipoise, the viscosity of the aqueous composition being measured at a pressure of 1.01325 10⁵ Pa, at a temperature of 22°C and at a shear rate of 1 s⁻¹.

2. The cosmetic product refill as claimed in claim 1, wherein the applicator member (26) has a density of between 0.11 and 0.15 g/cm³ and the aqueous composition has a viscosity greater than 20 centipoise and/or less than 25 centipoise, at 22°C.

3. The cosmetic product refill as claimed in claim 1 or 2, wherein the applicator member (26) is a felt, the fibers of the applicator member preferably being oriented parallel or perpendicularly to the surface intended to be in contact with the hair to be treated, and notably the fibers are oriented in a longitudinal direction of the applicator member (26), parallel to the surface intended to be in contact with the hair to be treated.

4. The cosmetic product refill as claimed in any one of claims 1 to 3, wherein the applicator member (26), notably the felt, if appropriate, is made of polyethylene, of polypropylene, or of a mixture of polyethylene and polypropylene, the mixture preferably comprising 50% polyethylene and 50% polypropylene.

5. The cosmetic product refill as claimed in any one of the preceding claims, wherein the applicator member (26) is soaked with aqueous composition with an initial degree of filling less than or equal to 90%, preferably less than or equal to 80%, more preferably less than or equal to 70%, and/or greater than or equal to 40%, preferably greater than or equal to 50%, more preferably greater than or equal to 60%.

6. The cosmetic product refill as claimed in one of the preceding claims, wherein the applicator member (26) is secured to a reservoir of aqueous composition to be applied to the hair to be treated, the reservoir preferably being made of the same material as the applicator member, more preferably with a lower density.

7. The cosmetic product refill as claimed in any one of the preceding claims, wherein the applicator member (26) and the reservoir, if appropriate, are saturated with a volume of aqueous composition greater than 6 ml, preferably greater than 10 ml, and/or less than 14 ml, preferably less than 11 ml.

8. The cosmetic product refill as claimed in any one of the preceding claims, wherein the applicator member (26) has an elongate shape, with a rectangular or cruciform cross section, with a flat, pointed or rounded end that is intended to be in contact with the hair to be treated, and/or with one or two bevels.

9. The cosmetic product refill as claimed in any one of the preceding claims, comprising a body delimiting a cavity that is formed at least partially by two opposite walls and a bottom and opens toward the outside by way of an opening, said applicator member (26) being partially received in said cavity and extending partially out of the cavity, through the opening.

10. The cosmetic product refill as claimed in any one of the preceding claims, wherein the aqueous composition comprises one or more non-silicone surfactants and/or one or more silicone compounds, the non-silicone surfactant(s) being chosen from anionic, cationic, nonionic or amphoteric surfactants, and preferably from cationic surfactants, the non-silicone surfactant(s) and/or the silicone compound(s) being preferably present in the aqueous composition in a content of between 0.01 and 10%, better still between 0.05 and 5% by weight, relative to the total weight of the aqueous composition.

11. The cosmetic product refill as claimed in any one of the preceding claims, wherein the aqueous composition comprises one or more fatty substances and/or one or more non-silicone polymers, the fatty substance(s) preferably being chosen from fatty alcohols, oils, and plant waxes, and the non-silicone polymer(s) preferably being chosen from cationic non-silicone polymers.

12. The cosmetic product refill as claimed in any one of the preceding claims, wherein the aqueous composition is in the form of an emulsion.

13. A cosmetic product refill article comprising a closed package and at least one cosmetic product refill as claimed in any one of the preceding claims, in the package.

14. A hair treatment device (1), preferably being a hair straightener, comprising:
- at least one holder, preferably an arm (5, 6) comprising a housing,
- a cosmetic product refill (20) as claimed in any one of claims 1 to 12, which is disposed in a removable manner in the housing.

15. A method for treating the hair, comprising the step of applying a cosmetic product to the hair using a device as claimed in claim 14.

## Patentansprüche

1. Nachfüllung (20) eines kosmetischen Produkts für ein Haarbehandlungsgerät (1), das einen Applikationsabschnitt (26) aufweist, bevorzugt einen porösen Applikationsabschnitt (26), der mit einer wässrigen Zusammensetzung gesättigt ist, wobei der Applikationsabschnitt eine Dichte von zwischen 0,11 und 0,25 g/cm³ hat, und **dadurch gekennzeichnet, dass**
die wässrige Zusammensetzung eine Viskosität zwischen 4 und 250 Centipoise, bevorzugt zwischen 4 und 100 Centipoise, hat, wobei die Viskosität von der wässrigen Zusammensetzung bei einem Druck von 1,01325 10⁵ Pa, bei einer Temperatur von 22°C und einer Scherrate von 1s⁻¹ gemessen worden ist.

2. Nachfüllung eines kosmetischen Produkts wie in Anspruch 1 beansprucht, wobei der Applikationsabschnitt (26) eine Dichte zwischen 0,11 und 0,15 Gramm pro Kubikzentimeter hat und die wässrige Zusammensetzung eine Viskosität von größer als 20 Centipoise und/oder weniger als 25 Centipoise bei 22°C hat.

3. Nachfüllung eines kosmetischen Produkts wie in Anspruch 1 oder 2 beansprucht, wobei der Applikationsabschnitt (26) ein Filz ist, wobei die Fasern von dem Applikationsabschnitt bevorzugt parallel oder senkrecht zu der Oberfläche orientiert sind, von der beabsichtigt ist, dass sie in Kontakt zu dem Haar, das zu behandeln ist, kommt, und insbesondere sind die Fasern in einer longitudinalen Richtung von dem Applikationsabschnitt (26) orientiert, parallel zu der Oberfläche, von der beabsichtigt ist, dass sie in Kontakt zu den Haaren, die zu behandeln sind, kommt.

4. Nachfüllung für ein kosmetisches Produkt wie in einem der Ansprüche 1 bis 3 beansprucht, wobei der Applikationsabschnitt (26), insbesondere das Filz, falls passend, aus Polyethylen, aus Polypropylen oder einer Mischung von Polyethylen und Polypropylen hergestellt ist, wobei die Mischung bevorzugt 50% Polyethylen und 50% Polypropylen aufweist.

5. Nachfüllung für ein kosmetisches Produkt wie in irgendeinem der voranstehenden Ansprüche beansprucht, wobei der Applikationsabschnitt (26) mit wässriger Zusammensetzung mit einem anfänglichen Füllgrad von weniger als oder gleich zu 90%, bevorzugt weniger als oder gleich zu 80%, noch bevorzugter weniger als oder gleich zu 70%, und/oder größer als oder gleich zu 40%, bevorzugt größer als oder gleich zu 50%, noch bevorzugter größer als oder gleich zu 60% getränkt ist.

6. Nachfüllung für ein kosmetisches Produkt wie in irgendeinem der voranstehenden Ansprüche beansprucht, wobei der Applikationsabschnitt (26) an einem Reservoir von wässriger Zusammensetzung befestigt ist, die auf das Haar anzuwenden ist, das zu behandeln ist, wobei das Reservoir bevorzugt aus dem gleichen Material hergestellt ist, wie der Applikationsabschnitt, noch bevorzugter mit einer geringeren Dichte.

7. Nachfüllung eines kosmetischen Produktes wie in irgendeinem der voranstehenden Ansprüche beansprucht, wobei der Applikationsabschnitt (26) und das Reservoir, falls passend, mit einem Volumen einer wässrigen Zusammensetzung von größer als 6 ml, bevorzugt größer als 10 ml, und/oder weniger als 14 ml, bevorzugt weniger als 11 ml gesättigt sind.

8. Nachfüllung eines kosmetischen Produkts wie in irgendeinem der voranstehenden Ansprüche beansprucht, wobei der Applikationsabschnitt (26) eine längserstreckte Form, mit einem rechtwinkligen oder kreuzförmigen Querschnitt, mit einem flachen, spitzen oder gerundeten Ende, das dazu gedacht ist, in Kontakt mit dem Haar, das zu behandeln ist, zu kommen, und/oder mit einer oder zwei Schrägen hat.

9. Nachfüllung eines kosmetischen Produkts wie in irgendeinem der voranstehenden Ansprüche beansprucht, die einen Körper hat, der eine Kavität begrenzt, die zumindest teilweise durch zwei gegenüberliegende Wandungen und einen Boden ausgebildet ist, und sich in Richtung der Außenseite mittels einer Öffnung öffnet, wobei der Applikationsabschnitt (26) teilweise in die Kavität aufgenommen ist und sich teilweise aus der Kavität durch die Öffnung erstreckt.

10. Nachfüllung eines kosmetischen Produkts wie in irgendeinem der voranstehenden Ansprüche beansprucht, wobei die wässrige Zusammensetzung aufweist, ein oder mehrere Nicht-Silikon-Tenside und/oder eine oder mehrere Silikonzusammensetzungen, wobei das Nicht-Silikon-Tensid (die Nicht-Silikon-Tenside) von anionischen, kationischen, nichtionischen oder amphoteren Tensiden, und bevorzugt von kationischen Tensiden, dem Nicht-Silikon-Tensid (den Nicht-Silikon-Tensiden) und/oder der Silikonzusammensetzung (den Silikonzusammensetzungen) ausgewählt ist, die bevorzugt in der wässrigen Zusammensetzung mit einem Inhalt von zwischen 0,01 und 10%, noch besser zwischen 0,05 und 5% relativ zu dem Gesamtgewicht der wässrigen Zusammensetzung zugegen sind.

11. Nachfüllung eines kosmetischen Produkts wie in irgendeinem der voranstehenden Ansprüche beansprucht, wobei die wässrige Zusammensetzung eine oder mehrere fettige Substanzen und/oder eine oder mehrere Nicht-Silikon-Polymere aufweist, wobei die fettige Substanz(en) bevorzugt von den fettigen Alkoholen, Ölen, und Pflanzenwachsen ausgewählt sind, und das Nicht-Silikon-Polymer (die Nicht-Silikon-Polymere) von den kationischen Nicht-Silikon-Polymeren bevorzugt ausgewählt sind.

12. Nachfüllung eines kosmetischen Produktes nach irgendeinem der voranstehenden Ansprüche, wobei die wässrige Zusammensetzung in der Form einer Emulsion vorliegt.

13. Nachfüllartikel für ein kosmetisches Produkt, das eine geschlossene Packung und zumindest eine Nachfüllung eines kosmetischen Produktes, wie in irgendeinem der voranstehenden Ansprüche beansprucht, in der Packung aufweist.

14. Haarbehandlungseinrichtung (1), die bevorzugt eine Haarglättungseinrichtung ist, die aufweist:
- zumindest einen Halter, bevorzugt einen Arm (5, 6), der ein Gehäuse aufweist,
- eine Nachfüllung (20) eines kosmetischen Produkts wie in irgendeinem der Ansprüche 1 bis 12 beansprucht, welche in einer entnehmbaren Weise in dem Gehäuse angeordnet ist.

15. Verfahren zur Behandlung des Haares, das die Schritte zum Anwenden eines kosmetischen Produkts auf das Haar unter Verwendung einer Einrichtung, wie in Anspruch 14 beansprucht, aufweist.

## Revendications

1. Recharge de produit cosmétique (20) pour dispositif de traitement de la chevelure (1), comportant un organe d'application (26), de préférence un organe d'application poreux (26), saturé au moyen d'une composition aqueuse, dans laquelle l'organe d'application présente une densité comprise entre 0,11 et 0,25 g/cm³ et **caractérisé en ce que** la composition aqueuse présente une viscosité comprise entre 4 et 250 centipoises, de préférence entre 4 et 100 centipoises, la viscosité de la composition aqueuse étant mesurée sous une pression de 1,01325 10⁵ Pa, à une température de 22°C et à une vitesse de cisaillement de 1 s⁻¹.

2. Recharge de produit cosmétique selon la revendication 1, dans laquelle l'organe d'application (26) a une densité comprise entre 0,11 et 0,15 g/cm³ et la composition aqueuse présente une viscosité supérieure à 20 centipoises et/ou inférieure à 25 centipoises, à 22°C.

3. Recharge de produit cosmétique selon la revendication 1 ou 2, dans laquelle l'organe d'application (26) est un feutre, les fibres de l'organe d'application étant de préférence orientées parallèlement ou perpendiculairement à la surface destinée à être en contact avec les cheveux à traiter, notamment les fibres sont orientées selon une direction longitudinale de l'organe d'application (26), parallèlement à la surface destinées à être en contact avec les cheveux à traiter.

4. Recharge de produit cosmétique selon l'une quelconque des revendications 1 à 3, dans laquelle l'organe d'application (26), notamment le feutre le cas échéant, est en polyéthylène, en polypropylène, ou en un mélange de polyéthylène et de polypropylène, le mélange comportant de préférence 50 % de polyéthylène et 50 % de polypropylène.

5. Recharge de produit cosmétique selon l'une quelconque des revendications précédentes, dans laquelle l'organe d'application (26) est imbibé de composition aqueuse avec un taux initial de remplissage inférieur ou égal à 90 %, de préférence inférieur ou égal à 80 %, de préférence encore inférieur ou égal à 70 %, et/ou supérieur ou égal à 40 %, de préférence supérieur ou égal à 50 %, de préférence encore supérieur ou égal à 60 %.

6. Recharge de produit cosmétique selon l'une des revendications précédentes, dans lequel l'organe d'application (26) est solidaire d'un réservoir de composition aqueuse à appliquer sur les cheveux à traiter, le réservoir étant de préférence réalisé dans le même matériau que l'organe d'application, de préférence encore avec une densité inférieure.

7. Recharge de produit cosmétique selon l'une quelconque des revendications précédentes, dans laquelle l'organe d'application (26) et le réservoir, le cas échéant, sont saturés d'un volume supérieur à 6 ml, de préférence supérieur à 10 ml, et/ou inférieur à 14 ml, de préférence inférieur à 11 ml de composition aqueuse.

8. Recharge de produit cosmétique selon l'une quelconque des revendications précédentes, dans laquelle l'organe d'application (26) a une forme allongée, de section transversale rectangulaire ou en croix, avec une extrémité destinée à être en contact avec les cheveux à traiter, plane, pointue ou arrondie, et/ou avec un ou deux biseaux.

9. Recharge de produit cosmétique selon l'une quelconque des revendications précédentes, comprenant un corps délimitant une cavité formée au moins partiellement par deux parois opposées et un fond, et s'ouvrant vers l'extérieur par une ouverture, ledit organe d'application (26) étant partiellement reçu dans ladite cavité et s'étendant en partie hors de la cavité, à travers l'ouverture.

10. Recharge de produit cosmétique selon l'une quelconque des revendications précédentes, dans laquelle la composition aqueuse comprend un ou plusieurs tensioactifs non siliconés et/ou un ou plusieurs composés siliconés, le ou les tensioactifs non siliconés étant choisis parmi les tensioactifs anioniques, cationiques, non ioniques ou amphotères et de préférence parmi les tensioactifs cationiques, le ou les tensioactifs non siliconés et/ou le ou les composés siliconés étant de préférence présents dans la composition aqueuse dans une teneur comprise entre 0,01 et 10%, mieux entre 0,05 et 5% en poids par rapport au poids total de la composition aqueuse.

11. Recharge de produit cosmétique selon l'une quelconque des revendications précédentes, dans laquelle la composition aqueuse comprend un ou plusieurs corps gras et/ou un ou plusieurs polymères non siliconés, le ou les corps gras étant choisis préférentiellement parmi les alcools gras, les huiles, et les cires végétales, et le ou les polymères non siliconés étant de préférence choisis parmi les polymères non siliconés cationiques.

12. Recharge de produit cosmétique selon l'une quelconque des revendications précédentes, dans laquelle la composition aqueuse est sous forme d'émulsion.

13. Article de recharge(s) de produit cosmétique, comprenant un emballage fermé et au moins une recharge de produit cosmétique selon l'une quelconque des revendications précédentes, dans l'emballage.

14. Dispositif de traitement de la chevelure (1), qui est de préférence un lisseur à cheveux, comportant :
- au moins un support, de préférence un bras (5, 6) comportant un logement,
- une recharge de produit cosmétique (20) selon l'une quelconque des revendications 1 à 12, disposée de façon amovible dans le logement.

15. Procédé de traitement de la chevelure comportant l'étape consistant à appliquer un produit cosmétique sur les cheveux à l'aide d'un dispositif selon la revendication 14.
